(19) Europäisches Patentamt
European Patent Office
Office européen des brevets

(11) **EP 1 889 924 B1**

(12) **EUROPEAN PATENT SPECIFICATION**

(45) Date of publication and mention of the grant of the patent:
**24.12.2014 Bulletin 2014/52**

(51) Int Cl.:
***C12Q 1/68*** (2006.01)

(21) Application number: **07102166.1**

(22) Date of filing: **12.02.2007**

(54) **Method of designing probes for detecting target sequence and method of detecting target sequence using the probes**

Verfahren zum Design von Sonden zum Nachweis von Zielsequenzen und Verfahren zum Nachweis von Zielsequenzen mittels Sonden

Procédé de conception de sondes pour détecter la séquence cible et procédé de détection de la séquence cible utilisant les sondes

(84) Designated Contracting States:
**DE GB**

(30) Priority: **17.08.2006 KR 20060077826**

(43) Date of publication of application:
**20.02.2008 Bulletin 2008/08**

(73) Proprietor: **Samsung Electronics Co., Ltd.**
**Suwon-si, Gyeonggi-do, 443-742 (KR)**

(72) Inventors:
• **Ahn, Tae-jin**
**Yongin-si**
**Gyeonggi-do (KR)**
• **Chung, Jong-suk**
**Yongin-si**
**Gyeonggi-do (KR)**
• **Lee, Jung-nam**
**Yongin-si**
**Gyeonggi-do (KR)**
• **Oh, Ji-young**
**Yongin-si**
**Gyeonggi-do (KR)**

(74) Representative: **Grünecker, Kinkeldey,**
**Stockmair & Schwanhäusser**
**Leopoldstrasse 4**
**80802 München (DE)**

(56) References cited:
**EP-A- 1 683 872      WO-A-01/11082**
**WO-A-97/46711**

• **DATABASE WPI Week 200413 Derwent Publications Ltd., London, GB; AN 2004-127112 XP002458262 & JP 2004 024247 A (KOKUSAI SHIYAKU KK) 29 January 2004 (2004-01-29)**
• **BODLEY TROUP, MARTIN, MCMILLAN, HORTON: "Simulated Pharmacogenomics Excercises for the Cybertory Virtual Molecular Biology Laboratory" PROCEEDINGS OF THE 2005 IEEE COMPUTATIONAL SYSTEMS BIOINFORMATICS CONFERENCE WORKSHOPS, 2005, XP002458261**
• **GUO Z ET AL: "ENHANCED DISCRIMINATION OF SINGLE NUCLEOTIDE POLYMORPHISMS BY ARTIFICIAL MISMATCH HYBRIDIZATION" NATURE BIOTECHNOLOGY, NATURE PUBLISHING GROUP, NEW YORK, NY, US, vol. 15, April 1997 (1997-04), pages 331-335, XP000867755 ISSN: 1087-0156**
• **WILSON KENNETH H ET AL: "High-density microarray of small-subunit ribosomal DNA probes", APPLIED AND ENVIRONMENTAL MICROBIOLOGY, AMERICAN SOCIETY FOR MICROBIOLOGY, US, vol. 68, no. 5, 1 May 2002 (2002-05-01), pages 2535-2541, XP002289520, ISSN: 0099-2240, DOI: 10.1128/AEM. 68.5.2535-2541.2002**

EP 1 889 924 B1

<110> Samsung Elecotronics Co. Ltd.

<120> Method of design probes for detecting target sequence and method of detecting target sequence using the probes

<130> EP47018FZ106

<140> not yet assigned
<141> herewith

<150> KR 10-2006-0077826
<151> 2006-08-17

<160> 17

<170> KopatentIn 1.71

<210> 1
<211> 60
<212> DNA
<213> streptococcus oralis

<400> 1

```
ctahgagatc gggacgccag tttcgaagga gacgttgttg ggatactacc cttgtgttat    60
                                                                     60
```

<210> 2
<211> 60
<212> DNA
<213> Streptococcus pneumoniae

<400> 2

```
ctaagagatc gggacgccag tttcgaagga gacgctgttg ggatactacc cttgtgttat    60
                                                                     60
```

<210> 3
<211> 60
<212> DNA
<213> Streptococcus dysgalactiae

<400> 3

```
cattgaagtc gggacgccag tttcgacgga ggcgttgttg ggatactacc cttgtgttat    60
                                                                     60
```

<210> 4
<211> 60
<212> DNA
<213> Streptococcus pyogenes

<400> 4

```
cattgacttc gggacgccag tttcgaatga ggcgttgttg ggatactacc cttgtgttat    60
                                                                     60
```

<210> 5

<211> 60
<212> DNA
<213> streptococcus sanguis

<400> 5

ctawgaratc gggacgccag tttcgatgga ggcgttgttg ggatactacc cttgtgttat          60

                                                                          60

<210> 6
<211> 60
<212> DNA
<213> Streptococcus bovis

<400> 6

ctatgaaatc gggacgctag tttcggtgga ggcgttgttg ggatactacc cttgtgttat          60

                                                                          60

<210> 7
<211> 60
<212> DNA
<213> streptococcus gordonii

<400> 7

ctacgagatc gggacgccag tttcgatgga ggcgctgttg ggatactacc cttgtgttat          60

                                                                          60

<210> 8
<211> 60
<212> DNA
<213> Gemella melitensis

<400> 8

ctaggaatca tgcacgccag tgtatgagga ggcgctgttg ggatactacc cttgctgtat          60

                                                                          60

<210> 9
<211> 17
<212> DNA
<213> Artificial Sequence

<220>
<223> perfect match probe #1

<400> 9
agtttcgaag gagacgt          17

<210> 10
<211> 25

<212> DNA
<213> Artificial sequence

<220>
<223> intended mismatch probe #1

<400> 10
agtttcgaag gagacgtagt tggga          25

<210> 11
<211> 17
<212> DNA
<213> Artificial Sequence

<220>
<223> perfect match probe #2

<400> 11
gtttcgaagg agacgtt          17

<210> 12
<211> 25
<212> DNA
<213> Artificial Sequence

<220>
<223> intended mismatch probe #2

<400> 12
gtttcgaagg agacgttctt gggat          25

<210> 13
<211> 17
<212> DNA
<213> Artificial Sequence

<220>
<223> perfect match probe #3

<400> 13
cgaaggagac gttgttg          17

<210> 14
<211> 25
<212> DNA
<213> Artificial sequence

<220>
<223> intended mismatch probe #3

<400> 14
cgaaggagac gttgttgcga tacta          25

<210> 15
<211> 17
<212> DNA
<213> Artificial sequence

<220>
<223> perfect match probe #4

<400> 15
aggagacgtt gttggga          17

<210> 16
<211> 25
<212> DNA
<213> Artificial sequence

<220>
<223> intended mismatch probe #4

<400> 16
aggagacgtt gttgggaaac taccc          25

<210> 17
<211> 20
<212> DNA
<213> Artificial sequence

<220>
<223> conventional probe

<400> 17
tttcgaagga gacgttgttg          20

**Description**

BACKGROUND OF THE INVENTION

1. Field of the Invention

The present invention relates to a method of preparing probes for detecting a target sequence and a method of detecting a target sequence using the probes.

2. Description of the Related Art

Due to recent advances in biotechnology, a base sequence formed of genetic information of individuals has been identified and thus research on a microarray for sequence analysis and disease diagnosis has widely conducted. A microarray is a substrate on which a group of polynucleotides is immobilized with high density, wherein each group of polynucleotides is immobilized at fixed locations. A method of analyzing a target sequence using a microarray is used to obtain a large amount of biological information, and a minimum amount of biomolecule sample such as DNA or protein is needed in the method.

A polynucleotide (also called "a probe", "a probe nucleic acid", or "a probe polynucleotide"), which is immobilized on the microarray, can be specifically hybridized with a target nucleic acid, and thus can be used to detect and identify the target sequence.

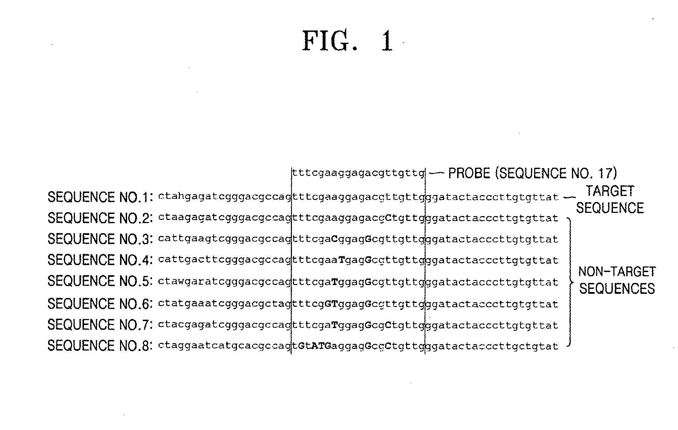

FIG. 1 is a schematic diagram illustrating an example of a conventional method of designing probes.

Referring to FIG 1, in the conventional method, one desirable probe sequence is selected, and the selected sequence can hybridize with the target sequence but does not cross-hybridize, i.e. hybridize with other, non-target sequences.

However, it is difficult to design a specific probe when sequence similarity between the target sequence and the non-target sequences is high or the number of target sequences to be identified is larger.

For example, in order to identify species of bacteria among a plurality of bacteria formed of a specific group of bacteria in a sample, a consensus sequence of a plurality of bacteria, in particular, specific sequences of a 16S rRNA site or 23S rRNA site, are used as probes. Such a method can be used to identify several species of bacteria, but is limited to the identification of ten or more species of bacteria since the sequence similarity is remarkably high.

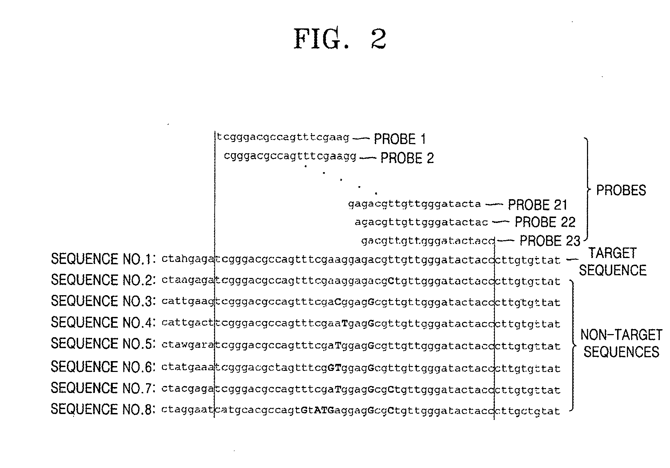

FIG. 2 is a schematic diagram illustrating another example of a conventional method of designing probes.

Referring to FIG. 2, in order to detect a target sequence, all possible probes having a defined length, which hybridize with, i.e. are completely complementary to a probe region in the target sequence are selected. The probe region in the target sequence is longer than the defined length of the probe. To cover the complete probe region of the target sequence, a plurality of probes are designed, sliding 1 bp by 1 bp along the probe region. The plurality of probes are disassembled at fixed locations.

However, since a large number of probes is used in this method, the result is hard to analyze and the manufacturing cost of DNA chips is high. Therefore, the consumption of time and effort is disadvantageous in such an analysis process.

WO 01/11082 A describes methods of monitoring expression of a plurality of genes in a cell or small population of cells. The method may entail contacting an array of probes with a population of nucleic acids derived from a population of fewer than 1000 cells, and then determining the relative hybridization of the probes of the population of nucleic acids as a measure of the relative representation of genes from the cells.

Bodley Troup, Martin, McMillan, Horton, Proceedings of the 2005 IEEE Computational Systems Bioinformatics Conferences Workshops, 2005, relates to simulated pharmacogenomics exercises for the cybertory virtual molecular biology laboratory. PCR products are genotyped by stimulated hybridization to an Affymetrix-style microarray testing for two single-nucleotide polymorphisms (SNP). Each SNP is represented by 4 rows of ten reporters. Each reporter is 25 nucleotides long and a first set of reporters represents perfect matches to a standard sequence, while the next ten reporters each contain a single artificial mismatch with respect to the standard sequence.

Guo, Z. et al. "Enhanced Discrimination of Single Nucleotide Polymorphisms by Artificial Mismatch Hybridization", Nature Biotechnology, Vol. 15, April 1997, pages 331-335, discloses inserting artificial mismatches into probe oligonucleotides using the base analog 3-nitropyrrole to increase the discrimination of single cell polymorphism in DNA hybridization.

Wilson, Kenneth et al., "High-density microarray of small-subunit ribosomal DNA probes", Applied and Environmental Microbiology, American Society for Microbiology, Vol. 68, No. 5, 1 May 2002, pages 2535-2541, refers to the use of photolithography chip technology to perform sequence analysis on amplified small subunit rRNA genes. The GeneChip contained 31,179 20-mer oligonucleotides that were complementary to a subalignment of sequences in the Ribosomal Database Project.

SUMMARY OF THE INVENTION

The present invention provides a method of designing and a method of preparing a probe for detecting a target sequence to rapidly and accurately detect the presence of the target sequence in reaction samples containing the target sequence and a large number of non-target sequences.

The present invention also provides a method of detecting a target sequence to rapidly and accurately detect the presence of the target sequence in reaction samples containing the target sequence and a large number of non-target sequences.

According to an aspect of the present invention, there is provided a method of preparing probes for detecting a target sequence, the method comprising: a) designing a matched probe and/or mismatched probe by: selecting an anchoring location in the target sequence, wherein the anchoring location is a nucleotide in the target sequence that is different from the corresponding nucleotide in a non-target sequence having the highest sequence similarity with the target sequence; selecting a first probe designing region, wherein the first probe designing region is a defined region in the target sequence, which defined region includes the anchoring location, wherein the first probe designing region is defined such that all probes designable as binding to the first designing region and having a defined length include the anchoring location, whereby the first probe designing region is represented by Formula 1:

$$< \text{Formula 1}>$$

$$i\text{-}n+1 \leq \text{first probe designing region} \leq i+n\text{-}1$$

wherein, i is an anchoring location and n is the length of the probe; selecting a probe selection location, wherein the probe selection location is a nucleotide in the target sequence that is different from the corresponding nucleotide in any of the non-target sequences, whereby the probe selection locations are included in a second probe designing region, wherein the second probe designing region is represented by Formula 2 when the length of the probe is an odd number, and is represented by Formula 3 when the length of the probe is an even number:

$$< \text{Formula 2}>$$

$$i\text{-}(n\text{-}1)/2 \leq \text{second probe designing region} \leq i+(n\text{-}1)/2$$

$$< \text{Formula 3}>$$

$$i\text{-}n/2+1 \leq \text{second probe designing region} \leq i+n/2\text{-}1,$$

wherein, i is an anchoring location and n is the length of a probe; and selecting a probe comprising (i) selecting a complete matched probe, wherein the complete matched probe includes a probe selection location at the center thereof and which complete matched probe has a sequence which enables complete hybridization of the complete matched probe with the target sequence; and (ii) selecting an intended mismatched probe including a probe selection location at a first location, a nucleotide which cannot hybridize with the corresponding nucleotide in the target sequence at a second location, and which intended mismatched probe has a sequence enabling, except for the second location, complete hybridization of the intended mismatched probe with the target sequence; and (b) synthesizing or isolating the matched probe and the mismatched probe as designed.

If there is one non-target sequence having the highest sequence similarity with the target sequence and if there are 2 or more possible anchoring locations in the target sequence with respect to said one non-target sequence, the anchoring location may be any one of said 2 or more possible anchoring locations.

If 2 or more non-target sequences have the same highest sequence similarity with the target sequence, the nucleotide in the target sequence, which is different from the highest number of corresponding nucleotides in the 2 or more non-target sequences having the same highest similarity with the target sequence, may be selected as the anchoring location.

If 2 or more non-target sequences have the same highest sequence similarity with the target sequence and if 2 or more nucleotides in the target sequence are different from the same highest number of corresponding nucleotides in the 2 or more non-target sequences having the same highest similarity with the target sequence, the anchoring location in the target sequence may be any one of said 2 or more nucleotides.

The center of the complete matched probe may be at (n+1) th when the length of the complete matched probe n is an odd number, and the center of the complete matched probe may be at any of n/2 th and n/2+1 th when the length of the complete matched probe n is an even number.

The first location may be at 1/3 of the intended mismatched probe, and the second location may be at 2/3 of the intended mismatched probe.

The locations where 1/3 and 2/3 of the intended mismatched probe may be at m/3 th and 2m/3+1 th, respectively, when the length m of the intended mismatched probe is a multiple of 3, descending integer of m/3 and (descending integer of m/3)×2+1 th, respectively, or (descending integer of m/3)+1 st and (descending integer of m/3)×2+2 th, respectively, when the length m of the intended mismatched probe is a multiple of 3+1, and (descending integer of m/3)+1 st and (descending integer of m/3)×2+2 nd, respectively, when the length m of the intended mismatched probe is a multiple of 3+2.

The length of the intended mismatched probe may be longer than the length of the complete matched probe. The length of the complete matched probe may be 17 to 25 bases and a length of the intended mismatched probe may be 25 to 36 bases.

According to another aspect of the present invention, there is provided a method of detecting a target sequence, the method comprising: preparing probes for detecting a target sequence according to any of the above described methods, wherein the step of selecting a probe comprises selecting a complete matched probe and selecting an intended mismatched probe; bringing a reaction sample into contact with the complete matched probe and the intended mismatched probe; and determining a hybridization reaction between the probes and the target sequences.

The method of detecting target sequence may further comprise manufacturing a microarray after selecting the complete matched probe and the intended mismatched probe, wherein the complete matched probe and the intended mismatched probe are fixed onto a substrate.

The method of detecting target sequence may further comprise a washing step after bringing the reaction sample into contact with the probes, wherein the reaction sample is substantially removed from the probes to eliminate a non-specific reaction.

Determining the hybridization reaction may be performed by detecting a fluorescence intensity and determining that hybridization is realized when the detected intensity is above a preset value.

The method of detecting a target sequence may further comprise determining the presence of the target sequence after determining the hybridization reaction, wherein the presence of the target sequence is determined using the result indicating whether hybridization or no hybridization is realized.

Determining the presence of the target sequence may be performed by comparing the result indicating whether hybridization or no hybridization is realized with a result of hybridization of reference samples only containing the target sequence to be detected.

The present invention also provides a computer readable recording medium having recorded thereon a program for performing any of the above described steps of designing probes for detecting target sequence.

The present invention also provides a microarray for the detection of a target sequence comprising a substrate and at least one probe that is fixed onto the substrate, characterized in that the at least one probe is prepared according to the method of preparing a probe for detecting a target sequence of the present invention; and that the matched probe and mismatched probe is fixed onto the substrate.

The microarray may preferably comprise at least one complete matched probe and at least one intended mismatched probe fixed onto the substrate.


BRIEF DESCRIPTION OF THE DRAWINGS

The above and other features and advantages of the present invention will all become more apparent by describing in detail exemplary embodiments thereof with reference to the attached drawings in which:

FIG. 1 is a schematic diagram illustrating an example of a conventional method of designing probes;
FIG. 2 is a schematic diagram illustrating another example of a conventional method of designing probes;
FIG. 3 is a flowchart illustrating a method of designing probes for detecting a target sequence according to an embodiment of the present invention;
FIG. 4A is a schematic diagram illustrating an example of selecting an anchoring location according to an embodiment of the present invention;
FIG. 4B is a schematic diagram illustrating a second example of selecting an anchoring location according to an embodiment of the present invention;
FIG. 4C is a schematic diagram illustrating a third example of selecting an anchoring location according to an embodiment of the present invention;
FIG. 5 is a schematic diagram illustrating an example of selecting a first probe designing region according to an embodiment of the present invention;

FIG. 6 is a schematic diagram illustrating an example of selecting a probe selection location, selecting a complete matched probe, and selecting an intended mismatched probe;

FIG. 7 is a flowchart of a method of detecting a target sequence according to an embodiment of the present invention; and

FIG. 8 is a schematic diagram illustrating an example of a determining the presence of a target sequence according to an embodiment of the present invention.

## DETAILED DESCRIPTION OF THE INVENTION

Hereinafter, the present invention will be described in more detail with reference to the accompanying drawings, in which exemplary embodiments of the invention are shown.

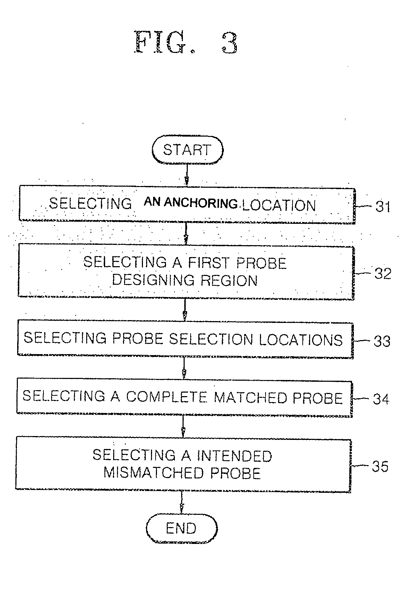

FIG. 3 is a flowchart illustrating a method of designing probes for detecting a target sequence according to a preferred embodiment of the present invention.

Referring to FIG. 3, the method of designing probes for detecting a target sequence includes selecting an anchoring location 31, selecting a first probe designing region 32, selecting probe selection locations 33, selecting a complete matched probe 34, and selecting an intended mismatched probe 35.

The preferred embodiment of Fig. 3 comprises both steps of selecting a complete matched probe 34 and selecting an intended mismatched probe 35. In an alternative embodiment, for example, if just a mismatched probe shall be designed, only one of selecting a complete matched probe 34 or selecting an intended mismatched probe 35 may be carried out, while the other step is omitted.

In the step of selecting the anchoring location 31, an anchoring location is chosen in the target sequence. The anchoring location is a nucleotide in the target sequence, which differs from the corresponding nucleotide in the non-target sequence having the highest sequence similarity with the target sequence to be detected. Methods for determining the sequence similarity of the target sequence and the non-target sequences in order to select the non-target sequence having the highest sequence similarity with the target sequence are known in the art.

As used herein, the term "the target sequence" refers to a polynucleotide selected to be detected by binding to a probe. Examples of the target sequence include genome DNA, a DNA fragment cleaved by a restriction enzyme, and a PCR product. A genome DNA fragment obtained by amplifying a specific region of genome DNA through a polymerase chain reaction (PCR) is generally used.

As used herein, the term "the non-target sequence" refers to all sequences other than the target sequence contained in the reaction samples. In the present invention, the detection of a target sequence and the presence of non-target sequences having very high sequence similarity is particularly considered.

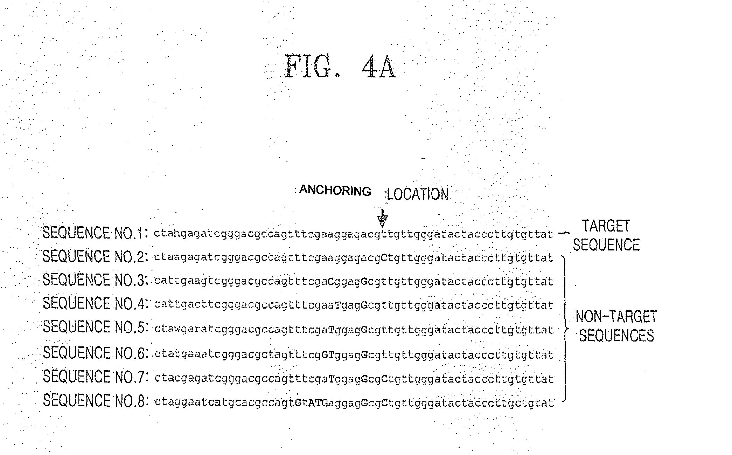

FIG. 4A is a schematic diagram illustrating an example of selecting an anchoring location according to an embodiment of the present invention.

Referring to FIG. 4A, one target sequence to be detected (sequence number 1) and 7 non-target sequences (sequence numbers 2 through 8) exist. Comparing the target sequence and the non-target sequences, the non-target sequence with sequence number 2 has a 1 base variation in comparison to the target sequence. The non-target sequences with sequence numbers 3 through 5 have 2 base variations, the non-target sequences with sequence numbers 6 and 7 have 3 base variations, and the non-target sequence with sequence number 8 has 6 base variations, all compared to the target sequence number 1. That is, the non-target sequence with sequence number 2 has the highest sequence similarity with the target sequence, and thus is taken into account when selecting the anchoring location. Considering the one base variation, where the target sequence is different form the non-target sequence number 2 having the highest similarity with sequence number 1, the target sequence has nucleotide "t", while the non-target sequence with sequence number 2 has nucleotide "c" at the corresponding location. The location of the target sequence indicating nucleotide "t", i.e. the nucleotide where the target sequence is different form the non-target sequence having the highest similarity with the target sequence, is thus selected as the anchoring location.

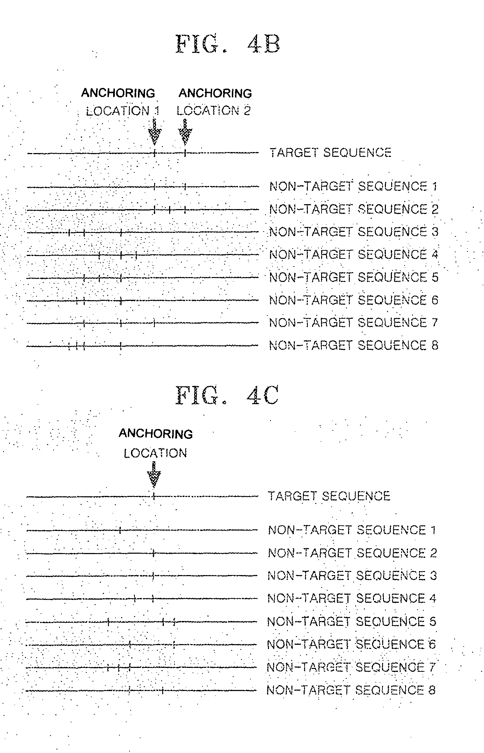

FIG. 4B is a schematic diagram illustrating another example of selecting an anchoring location according to an embodiment of the present invention.

Referring to FIG. 4B, the non-target sequence 1 has the highest homology with the target sequence. The target sequence and the non target sequence 1 show a two base variance, i.e. differ at two different locations compared from one another. The vertical bars crossing the horizontal lines corresponding to the sequences indicate differences between the target sequence and a non-target sequence. As can be seen in Fig. 4B, the non-target sequence 1, which has the highest similarity with the target sequence, shows a 2 nucleotide variation, i.e. differs at two locations with respect to the target sequence.

According to the embodiment shown in Fig. 4B, when the non-target sequence having the highest sequence similarity with the target sequence comprises two or more base variations, i.e. nucleotides, which differ from the corresponding nucleotides of the target sequence, any one of the base variations can be selected as the anchoring location. In other words, if there is one non-target sequence having the highest sequence similarity with the target sequence and if there are at least two possible anchoring locations, any one of the possible anchoring locations may be selected.

In FIG. 4B, any one of the two possible anchoring locations 1 and 2 can be selected as an anchoring location.

FIG. 4C is a schematic diagram illustrating another example of selecting an anchoring location according to a further embodiment of the present invention.

Referring to FIG. 4C, the non-target sequences 1 to 3 all have the same highest sequence similarity with the target sequence, namely a one base variation in comparison with the target sequence. Thus, the non-target sequence having the highest sequence similarity with the target sequence corresponds to 2 or more non-target sequences having the same highest sequence similarity with the target sequence in the embodiment shown in Fig. 4C. In such a case, the nucleotide in the target sequence, which is different from the highest number of corresponding nucleotides in the 2 or more non-target sequences having the same highest similarity with the target sequence, is selected as the anchoring location.

This can be done by grouping these non-target sequences having the same highest sequence similarity with the target sequence according to the location, where the base variation with regard to the target sequence occurs. In other words, the non-target sequence 1 (constituting group 1) is different from the target sequence at a first varying location in the target sequence 1 (group 1). The non-target sequences 2 and 3 (constituting group 2) are each different from the target sequence at a second varying location, which is different from the first varying location. The non-target sequences of the groups 1 and 2 have all the same homology with the target sequence, but the base variation(s) of said non-target sequences occur in different frequencies. The frequency is defined as the number of non-target sequences comprising a specific base variation with respect to the total number of non-target sequences having the highest sequence similarity with the target sequence. The nucleotide in the target sequence that is different from the most of the corresponding nucleotides in the 2 or more non-target sequences having the highest similarity with the target sequence is selected as anchoring location. In other words, the variation of the target sequence having the highest frequency among all of the non-target sequences having the highest homology with the target sequence is selected as anchoring location. In this case, the varying location in the target sequence, which is different from the corresponding location of the non-target sequences constituting group 2 is selected as an anchoring location.

On the other hand, when 2 or more non-target sequences having the same highest sequence similarity with the target sequence exist and 2 or more base variation locations of these non-target sequences have the same highest frequency, any one of said base variation locations in the target sequence, which is different from the non-target sequences having the same highest sequence similarity with the target sequence, can be selected as an anchoring location. In other word, if 2 or more non-target sequences have the same highest sequence similarity with the target sequence and if 2 or more nucleotides in the target sequense are different from the same highest number of corresponding nucleotides in the 2 or more non-target sequences having the same highest similarity with the target sequence, the anchoring location in the target sequence is any one of said 2 or more nucleotides. In the step of selecting the first probe designing region 32, the first designing region is a fixed region in the target sequence including the anchoring location and having a defined length. Preferably, the first probe designing region is selected such that all probes designable as binding to the first designing region and having a defined length include the anchoring location.

The first probe designing region may be represented by Formula 1.

<Formula 1>

$$i-n+1 \leq \text{first probe designing region} \leq i+n-1$$

wherein, i is the anchoring location and n is the length of the probe.

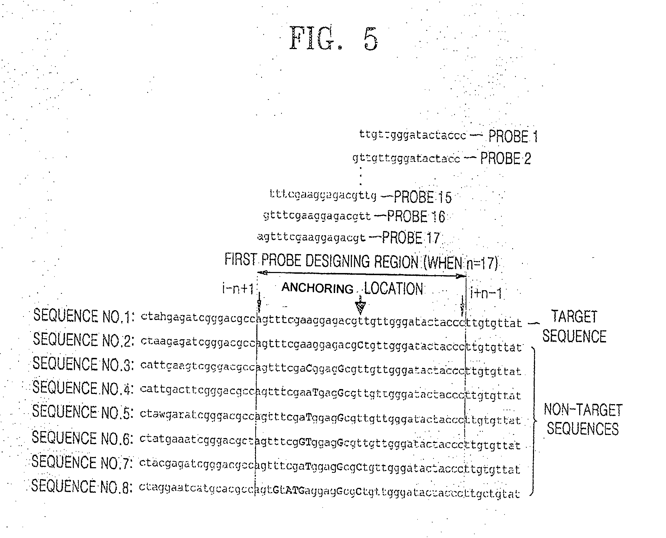

FIG. 5 is a schematic diagram illustrating an example of selecting a first probe designing region according to an embodiment of the present invention.

In practice, a probe having the a nucleotide sequence that is complementary with the target sequence can hybridize with the target sequence. In other words, hybridization of A-T and C-F substantially occurs. For intuitive understanding, however, it is expressed herein that hybridization occurs between the same nucleotide such as A-A and C-C.

Referring to FIG. 5, the defined length of the probe is set to 17 bases. In this case, the number of probes including the anchoring location that can be designed is 17. The probes 1 through 17 are designed in the first probe designing region, wherein all said probes 1 to 17 include the anchoring location.

When n=17 and i=0 are introduced in Formula 1, the first probe designing region is in a range of -16 to 16, downstream and upstream the anchoring location in the target sequence.

In the step of selecting the probe selection location(s) 33, a second probe designing region is selected, and the probe selection location(s) included in the second probe designing region is/are selected in the target sequence. A probe selection location is a nucleotide in the target sequences, which is different from the corresponding nucleotide in any of the non-target sequences.

The second probe designing region is included in the first probe designing region and comprises the target sequence in the range starting in the center portion of the probe 1 and ending in the center portion of the probe 17 as illustrated in FIG. 5. This is, the second probe designing region extends from the middle of probe 1, which comprises the sequence ranging from the first nucleotide of the first probe designing region to the anchoring location, up to the middle of probe "n", here probe 17, which comprises the sequence ranging from the anchoring location to the last nucleotide of the first probe designing region.

The second probe designing region may be represented by Formula 2, when the length of the probe is an odd number, and may be represented by Formula 3, when the length of the probe is an even number.

$$<\text{Formula 2}>$$

$$i-(n-1)/2 \leq \text{second probe designing region} \leq i+(n-1)/2$$

$$<\text{Formula 3}>$$

$$i-n/2+1 \leq \text{second probe designing region} \leq i+n/2-1$$

wherein, i is an anchoring location and n is a length of a probe.

Since the probe selection location is selected in the second probe designing region, a complete matched probe can include an anchoring location.

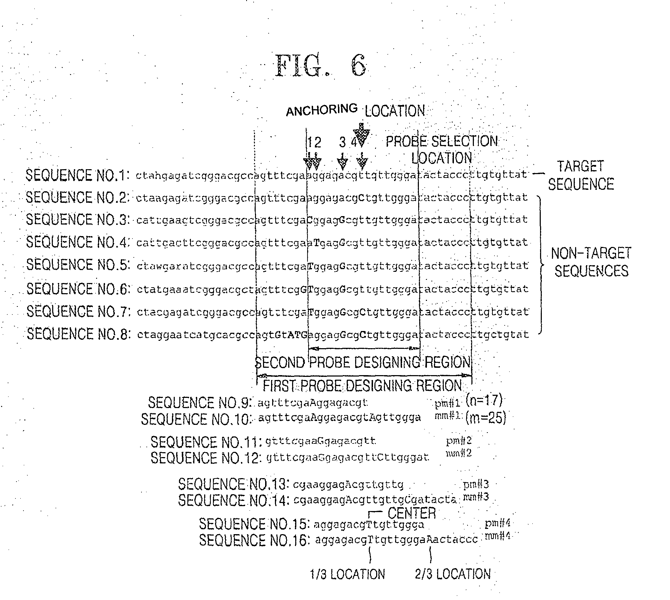

FIG. 6 is a schematic diagram illustrating an example of selecting a probe selection location 33, selecting a complete matched probe 34, and selecting an intended mismatched probe 35.

Referring to FIG. 6, the second probe designing region, when the length of the probe is n=17, and the probe selection locations 1 through 4, i.e. the nucleotides in the second probe designing region, which are different in the target sequence compared to the corresponding nucleotide in any of the non-target sequences, that represent are illustrated.

The predefined length of the probe is n=17, i.e. an odd number. The second probe designing region can be accordingly calculated using Formula 2. When n=17 and i=0 are introduced in Formula 2, the second probe designing region is in a range of -8 to 8, downstream and upstream the anchoring location in the target sequence as indicated in Fig. 6.

Next, the probe selection locations included in the second probe designing region in the target sequence are selected, wherein a probe selection location is a nucleotide in the target sequences, which is different from the corresponding nucleotide in any of the non-target sequences. In the embodiment of Fig. 6, 4 probe selection locations, including the anchoring location, are present within the second probe designing region.

In the step of selecting a complete matched probe 34, a complete matched probe is selected having a sequence enabling complete hybridization with the target sequence, wherein said probe includes a probe selection location at the center thereof.

When the length n of the complete matched probe is an odd number, the center of the complete matched probe is at (n+1)/2 th, and when the length n of the complete matched probe is an even number, the center of the complete matched probe is at any of n/2 th and n/2+1 th.

In the step of selecting an intended mismatched probe 35, an intended mismatched probe is selected including the probe selection location at first location, a nucleotide which cannot hybridize with the target sequence at second location, and a sequence enabling, except for the second location, complete hybridization with the target sequence. Preferably, the intended mismatched probe is longer than the complete matched probe, and most preferably, the intended mismatched probe comprises a complete matched probe selected as described above.

The first location may be at 1/3 of the intended mismatched probe and the second location may be at 2/3 of the intended mismatched probe.

When the direction is set from 5' to 3', the first and the second location may be at 1/3 and 2/3 of the intended mismatched probe, respectively, or may be at 2/3 and 1/3 of the intended mismatched probe.

When the length m of the intended mismatched probe is a multiple of 3, 1/3 and 2/3 of the intended mismatched probe are m/3 th and 2m/3+1 th, respectively. When the length m of the intended mismatched probe is a multiple of 3+1, 1/3 and 2/3 of the intended mismatched probe are descending integer of m/3 and (descending integer of m/3)x2+1 th, respectively, or (descending integer of m/3)+1 st and (descending integer of m/3)$\times$2+2 th, respectively. When the length m of the intended mismatched probe is a multiple of 3+2, 1/3 and 2/3 of the intended mismatched probe may be (descending integer of m/3)+1 st and (descending integer of m/)$\times$2+2 nd, respectively. Descending integer of m/3 refers to the quotient of m/3 that is then rounded off.

The length of the complete matched probe may preferably be 17 to 25 bases and the length of the intended mismatched probe may be 25 to 36 bases, but is not limited thereto. The length of the complete matched probe may be 2/3 times of the intended mismatched probe.

Referring to FIG. 6, the complete matched probe (pm) and the intended mismatched probe (mm) are designed with respect to the probe selection locations 1 through 4. The length n of the complete matched probe (pm) and the length m of the intended mismatched probe (mm) is set to 17 and 25, respectively.

For example, the complete matched probe pm #4 (sequence No. 15) is designed with respect to the probe selection location 4. Probe pm #4 has a length of 17 bases, a sequence enabling complete hybridization with the target sequence and includes the probe selection location 4 at the center thereof. The complete matched probe pm #4 (sequence number 15) can completely hybridize with the target sequence (sequence number 1) and can incompletely hybridize with the non-target sequence with sequence number 2 due to the 1 base variation at the probe selection location 4.

In addition, the intended mismatched probe mm #4 (sequence number 16) is designed with respect to the probe selection location 4. The intended mismatched probe mm #4 comprises 25 bases, thus is longer than the complete matched probe pm #4. Mm #4 includes the probe selection location 4 at first location, a nucleotide, which cannot hybridize with the target sequence at a second location, and a sequence enabling, except for the second location, complete hybridization with the target sequence. The probe selection location 4 is at 1/3 of the intended mismatched probe mm #4 (sequence number 16) and the nucleotide, which cannot hybridize with the target sequence, is at 2/3 of the intended mismatched probe mm #4 (sequence number 16). The intended mismatched probe mm #4 (sequence number 16) can incompletely hybridize with the target sequence (sequence number 1) due to the at least 1 base variation at 2/3. However, intended mismatched probe mm #4 cannot hybridize with the non-target sequence with sequence number 2 at two nucleotides due to the at least 2 base variations at the probe selection location 4 and at the nucleotide which cannot hybridize with the target sequence at 2/3 of the intended mismatched probe mm #4.

The nucleotide in the target sequence corresponding to the location at 1/3 of the intended mismatched probe mm #1 is "t" and the nucleotide in the target sequence corresponding to the location at 2/3 of the intended mismatched probe (mm #1) is "a". In addition, the nucleotide at 2/3 of the intended mismatched probe (mm #1) may likewise be "c" or "g" since the target sequence comprises nucleotide "t" at the corresponding location.

The method of designing probes for detecting a target sequence according to an embodiment of the present invention can be embodied as computer readable codes (including all devices having an Information processing function) on a computer readable recording medium, e.g. a computer-readable medium having computer-executable instructions for performing the method of designing probes for detecting a target sequence according to an embodiment of the present invention. The computer readable recording medium is any data storage device that can store data which can be thereafter read by a computer system. Examples of the computer readable recording medium include read-only memory (ROM), random-access memory (RAM), CD-ROMs, magnetic tapes, floppy disks, and optical data storage devices. The devices having an information processing function can be any device having means for performing the method of designing probes for detecting a target sequence according to an embodiment of the present invention, and may preferably be a computer system having a memory, an operating system and a central processor, the computer system operable to carry out the steps of the method of designing probes for detecting a target sequence according to an embodiment of the present invention.

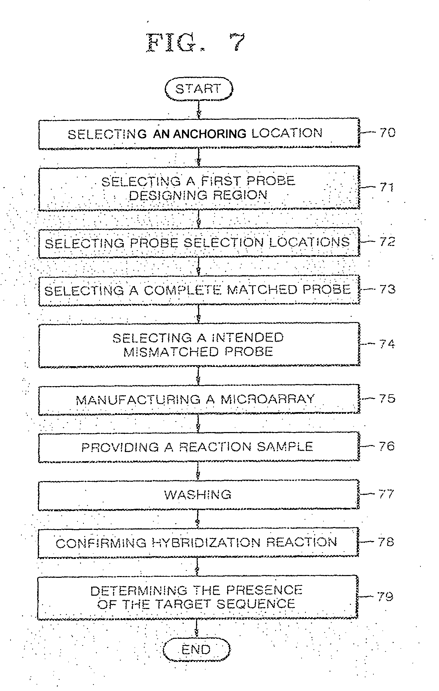

FIG. 7 is a flowchart of the method of detecting a target sequence according to an embodiment of the present invention.

Referring to FIG. 7, the method of detecting target sequence includes selecting an anchoring location 70, selecting a first probe designing region 71, selecting probe selection locations 72, selecting a complete matched probe 73, selecting a intended mismatched probe 74, manufacturing a microarray 75, providing a reaction sample, washing 77, determining a hybridization reaction 78, and determining the presence of the target sequence 79.

The operation of selecting the anchoring location 70, selecting the first probe designing region 71, selecting the probe selection locations 72, selecting the complete matched probe 73, and selecting the intended mismatched probe 74 correspond to the respective steps 31 to 35 in the method of designing probes for detecting a target sequence described above and thus a detailed description thereof is omitted.

In the step of manufacturing the microarray 75, a microarray for detecting a target sequence is manufactured, wherein the microarray comprises a substrate, at least one complete matched probe and at least one intended mismatched probe, which probes are designed according to any the above described method of designing probes for detecting a target sequence and which probes are fixed onto a substrate of the microarray.

The microarray may be manufactured by fixing the designed and synthesized probes onto the substrate any conventional method known to those skilled in the art. That is, the substrate may be coated with an active group selected from the group consisting of amino-silane, poly-L-lysine, and aldehyde. The substrate may be a silicon wafer, glass, quartz, metal, or plastic. The probe set may be immobilized on the substrate using a piezoelectric micropipetting method, a pin-shaped spotter, and etc.

In the step of providing the reaction sample 76, a reaction sample is brought into contact with the complete matched probe(s) and the intended mismatched probe(s), which are preferably immobilized onto the microarray substrate, for hybridizing the probe(s) with the components of the reaction sample in order to detect the presence of a target sequence in the reaction sample. In other words, the reaction sample(s) may be provided to the probes of the microarray manufactured as described above in a way allowing a hybridization reaction to progress.

In the washing step 77, the reaction sample(s) are substantially removed, e.g. washed off the microarray, to eliminate any non-specific reaction.

In the step of confirming hybridization reaction 78, any hybridization reaction between one of the probes and the corresponding target sequence is confirmed. The determination if hybridization occurred can be performed by detecting a fluorescence intensity and determining that the hybridization is realized when the fluorescence intensity measured is above a predetermined standard value.

An example of determining hybridization by detecting the fluorescence intensity with respect to different probes is illustrated in Table 1.

<Table 1 >

|  | pm #1 | mm #1 | pm #2 | mm #2 | pm #3 | mm #3 | pm #4 | mm #4 |
|---|---|---|---|---|---|---|---|---|
| Intensity | 10020 | 9479 | 5977 | 27259 | 5365 | 831 | 24911 | 9459 |

In the example illustrated in Table 1, for example, the predetermined standard or threshold value may be set at the fluorescence intensity of 3000, that is any fluorescence intensity above 3000 indicates that a hybridization is realized at the respective probe. In other words, it is determined as "ON." On the contrary, when the fluorescence intensity is below 3000, hybridization is not realized. In other words, it is determined as "OFF."

In another method, the predetermined standard value may be calculated by Formula 4 and hybridization is realized if the value according to Formula 4 is above 3. In other words, it is determined as "ON." On the contrary, when the value of Formula 4 is below 3, hybridization is not realized. In other words, it is determined as "OFF."

<Formula 4>

$$\log_2[(\text{fluorescence intensity of each probe})/(\text{background intensity})]$$

In the above Formula 4, the background intensity is the fluorescence intensity when reference samples in which nucleic acid is not included are provided.

Table 2 illustrates an example of values obtained when the fluorescence intensity of each probe is divided by specific background intensity according to Formula 4.

<Table 2>

|  | pm #1 | mm #1 | pm #2 | mm #2 | pm #3 | mm #3 | pm #4 | mm #4 |
|---|---|---|---|---|---|---|---|---|
| Log data | 3.47 | 3.54 | 4.21 | 3.02 | 2.32 | 3.42 | 2.92 | 3.74 |

In Table 3, when a value of Table 2 is above 3, "ON" is indicated and when a value of Table 2 is below 3, "OFF" is indicated.

<Table 3>

|  | pm #1 | mm #1 | pm #2 | mm #2 | pm #3 | mm #3 | pm #4 | mm #4 |
|---|---|---|---|---|---|---|---|---|
| Log data | ON | ON | ON | ON | OFF | ON | OFF | ON |

In the step of determining the presence of the target sequence 79, the presence of the target sequence is determined using the result indicating whether hybridization is realized or not at the probes of the microarray. The determining of the presence of the target sequence may be performed by comparing the result above, i.e. a test result, with a result of hybridization of reference samples only containing the target sequence.

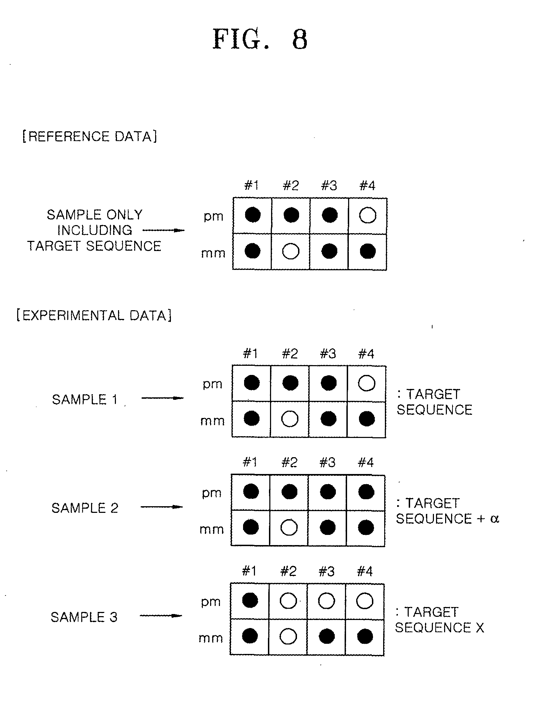

FIG. 8 is a schematic diagram illustrating an example of determining the presence of the target sequence according to an embodiment of the present invention.

Referring to FIG. 8, samples only including the target sequence are provided to the microarray and then reference data is obtained as described above. In the reference data, pm #1, pm #2, pm #3, mm #1, mm #3, and mm #4 become "ON", while pm #4 and mm #2 are confirmed "OFF."

A sample 1 for detecting the presence of the target sequence is provided to the same microarray to obtain experimental data. The experimental data of the sample 1 is the same with the reference data and thus it is concluded that the target sequence exists in the sample 1.

In the experimental data obtained by providing a sample 2 to the microarray, pm #4 is "ON" in addition to probes pm #1, pm #2, pm #3, mm #1, mm #3, and mm #4 indicating "ON" in the reference data. Therefore, it is concluded that sample 2 includes the target sequence and other sequences.

In the experimental data obtained by providing a sample 3 to the microarray, only the probes pm #1, mm #1, mm #3, and mm #4 are "ON." The experimental data of the sample 3 is different from the reference data and thus it can be concluded that the target sequence is not included in the sample 3.

The present invention will be described in greater detail with reference to the following examples. The following examples are for illustrative purposes and are not intended to limit the scope of the invention.

Example 1

Designing probes for detecting a target sequence according to the present invention

As illustrated in FIG. 6, 4 complete matched probes and 4 intended mismatched probes (sequence numbers 9 through 16) which can specifically detect the target sequence, the 23S-rRNA of *Streptococcus oralis* (*sor*) (sequence number 1) from the non-target sequences (sequence numbers 2 through 8) were designed.

Sequences with sequence numbers 2 through 8 are corresponding regions of 23S-rRNA in *Streptococcus pneumoniae* (*spn*) (sequence number 2), *Streptococcus dysgalactia* (*sgo*) (sequence number 3), *Streptococcus pyogenes* (*sdy*) (sequence number 4), *Streptococcus sanguinis* (*spo*) (sequence number 5), *Streptococcus bovis* (*ssu*) (sequence number 6), *Streptococcus Gordonii* (*sbo*) (sequence number 7), and *Gemella melitensis* (*gme*) (sequence number 8).

Comparative Example 1

Designing probes for detecting a target sequence according to a conventional method

As illustrated in FIG. 1, the probe (sequence number 17) for detecting the target sequence (sequence number 1) was designed using the conventional method.

Experimental Example 1

Detecting the target sequence

The probes (sequence number 9 through 16) according to the present invention and the conventional probe (sequence number 17) were used to determine in 16 test samples whether the target sequence can be specifically detected and the results were compared.

The results are shown in Table 4. As illustrated in Table 4, the target sequence is accurately detected in 11 experiments among the 16 experiments using the conventional method, and the target sequence is accurately detected in 15 experiments using the method according to the present invention.

<Table 4>

| samples | correct | conventional method | pm#1 | pm #2 | pm #3 | pm #4 | present invention | improvement |
|---|---|---|---|---|---|---|---|---|
| | | | mm #1 | mm #2 | mm #3 | mm #4 | | |
| sor | sor | sor | ○ ○ | ○ ○ | ○ ○ | ○ x | sor | |
| spn | non-sor | sor | ○ ○ | ○ ○ | ○ x | ○ x | non-sor | improved |

(continued)

| samples | correct | conventional method | pm#1 | pm #2 | pm #3 | pm #4 | present invention | improvement |
|---------|---------|---------------------|------|-------|-------|-------|-------------------|-------------|
|  |  |  | mm #1 | mm #2 | mm #3 | mm #4 |  |  |
| sdy | non-sor | non-sor | x<br>x | x<br>○ | x<br>x | ○<br>x | non-sor |  |
| SSU | non-sor | non-sor | x<br>x | x<br>x | x<br>x | x<br>x | non-sor |  |
| sbo | non-sor | non-sor | x<br>x | x<br>x | x<br>x | x<br>x | non-sor |  |
| sgo | non-sor | non-sor | x<br>x | x<br>x | x<br>x | x<br>x | non-sor |  |
| gme | non-sor | non-sor | x<br>x | x<br>x | x<br>x | x<br>x | non-sor |  |
| spn+gme | non-sor | sor | ○<br>○ | x<br>○ | ○<br>x | ○<br>○ | non-sor | improved |
| spn+sor | sor+alpha | sor | ○<br>○ | ○<br>○ | ○<br>○ | ○<br>x | sor |  |
| spn+ssu | non-sor | sor | ○<br>○ | x<br>○ | ○<br>x | ○<br>x | non-sor | improved |
| spn+gme+sor | sor+alpha | sor | ○<br>○ | ○<br>○ | ○<br>○ | ○<br>○ | sor+alpha |  |
| spn+gme+ssu | non-sor | sor | ○<br>x | x<br>○ | x<br>x | ○<br>○ | non-sor | improved |
| spn+sor+ssu | sor+alpha | sor | ○<br>○ | ○<br>○ | ○<br>○ | ○<br>x | sor |  |
| spn+gme+sor+ssu | sor+alpha | sor | ○<br>○ | ○<br>○ | ○<br>○ | ○<br>x | sor |  |
| gme+ssu | non-sor | sor | ○<br>○ | ○<br>○ | ○<br>x | ○<br>x | non-sor | improved |
| sor+ssu | sor+alpha | sor | ○<br>○ | ○<br>○ | ○<br>x | ○<br>x | non-sor | retrograded |

According to the method of preparing probes for detecting a target sequence of the present invention, probes can be designed to rapidly and accurately detect the presence of the target sequence in reaction samples containing the target sequence and a large number of non-target sequences.

In addition, according to the method of detecting target sequence of the present invention, the presence of the target sequence is rapidly and accurately detected in reaction samples containing the target sequence and a large number of non-target sequences.

While the present invention has been particularly shown and described with reference to exemplary embodiments thereof, it will be understood by those of ordinary skill in the art that various changes in form and details may be made therein without departing from the scope of the present invention as defined by the following claims

## Claims

1. A method of preparing probes for detecting a target sequence, the method comprising:

   a. designing a matched probe and/or a mismatched probe by:

   selecting an anchoring location in the target sequence, wherein the anchoring location is a nucleotide in the target sequence that is different from the corresponding nucleotide in a non-target sequence having the highest sequence similarity with the target sequence;
   selecting a first probe designing region, wherein the first probe designing region is a defined region in the target sequence, which defined region includes the anchoring location, wherein the first probe designing region is defined such that all probes designable as binding to the first probe designing region and having a defined length include the anchoring location, wherein the first probe designing region is represented by Formula 1:

$$< \text{Formula 1} >$$
$$i-n+1 \leq \text{first probe designing region} \leq i+n-1$$

   wherein, i is an anchoring location and n is the length of the probe;
   selecting a probe selection location, wherein the probe selection location is a nucleotide in the target sequence that is different from the corresponding nucleotide in any of the non-target sequences, wherein the probe selection location is included in the first probe designing region, wherein the probe selection locations are included in a second probe designing region, and wherein the second probe designing region is represented by Formula 2 when the length of the probe is an odd number, and is represented by Formula 3 when the length of the probe is an even number:

< Formula 2>

i-(n-1)/2 ≤ second probe designing region ≤ i+(n-1)/2

< Formula 3>

i-n/2+1 ≤ second probe designing region ≤ i+n/2-1,

wherein, i is an anchoring location and n is the length of a probe; and
selecting a probe comprising (i) selecting a complete matched probe, wherein the complete matched probe includes the probe selection location at the center thereof and which complete matched probe has a sequence which enables complete hybridization of the complete matched probe with the target sequence; and (ii) selecting an intended mismatched probe including the probe selection location at a first location, a nucleotide which cannot hybridize with the corresponding nucleotide in the target sequence at a second location, and which intended mismatched probe has a sequence enabling, except for the second location, complete hybridization of the intended mismatched probe with the target sequence; and

b. synthesizing or isolating the matched probe and mismatched probe as designed.

2. The method of claim 1, wherein, if there is one non-target sequence having the highest sequence similarity with the target sequence and if there are 2 or more possible anchoring locations in the target sequence with respect to said one non-target sequence, the anchoring location is any one of said 2 or more possible anchoring locations.

3. The method of claim 1, wherein, if 2 or more non-target sequences have the same highest sequence similarity with the target sequence, the nucleotide in the target sequence, which is different from the highest number of corresponding nucleotides in the 2 or more non-target sequences having the same highest similarity with the target sequence, is selected as the anchoring location.

4. The method of claim 1, wherein, if 2 or more non-target sequences have the same highest sequence similarity with the target sequence and if 2 or more nucleotides in the target sequence are different from the same highest number of corresponding nucleotides in the 2 or more non-target sequences having the same highest similarity with the target sequence, the anchoring location in the target sequence is any one of said 2 or more nucleotides.

5. The method of any of claims 1 to 4, wherein the center of the complete matched probe is at (n+1)/2 th when the length n of the complete matched probe is an odd number, and the center of the complete matched probe is at any of n/2 th and n/2+1 th when the length n of the complete matched probe is an even number.

6. The method of any of claims 1 to 5, wherein the first location is at 1/3 of the intended mismatched probe, and the second location is at 2/3 of the intended mismatched probe.

7. The method of claim 6, wherein 1/3 and 2/3 of the intended mismatched probe are at m/3 th and 2m/3+1 th, respectively, when the length m of the intended mismatched probe is a multiple of 3, descending integer of m/3 and (descending integer of m/3)×2+1 th, respectively, or (descending integer of m/3)+1 st and (descending integer of m/3)×2+2 th, respectively, when the length m of the intended mismatched probe is a multiple of 3+1, and (descending integer of m/3)+1 st and (descending integer of m/3×2+2 nd, respectively, when the length m of the intended mismatched probe is a multiple of 3+2.

8. The method of any of claims 1 to 7, wherein the intended mismatched probe is longer than the complete matched probe,

9. The method of claim 8, wherein the length n of the complete matched probe is 17 to 25 bases and the length m of the intended mismatched probe is 25 to 36 bases.

10. A method of detecting a target sequence, the method comprising:

preparing probes for detecting a target sequence according to any of the methods set forth in claims 1 to 9, wherein the step of selecting a probe comprises selecting a complete matched probe and selecting an intended mismatched probe;

bringing a reaction sample into contact with the complete matched probe and the intended mismatched probe; and

determining a hybridization reaction between the probes and the target sequences.

11. The method of claim 10, further comprising manufacturing a microarray after selecting the complete matched probe and the intended mismatched probe, wherein the complete matched probe and the intended mismatched probe are fixed onto a substrate.

12. The method of claim 10 or 11, further comprising a washing step after bringing the reaction sample into contact with the probes, wherein the reaction sample is substantially removed from the probes to eliminate a non-specific reaction.

13. The method of any of claims 10 to 12, wherein determining the hybridization reaction is performed by detecting fluorescence intensity and determining that hybridization is realized when the detected intensity is above a preset value.

14. The method of any of claims 10 to 13, further comprising determining the presence of the target sequence after determining the hybridization reaction, wherein the presence of the target sequence is determined using the result indicating whether hybridization or no hybridization is realized.

15. The method of claim 14, wherein determining the presence of the target sequence is performed by comparing the result indicating whether hybridization or no hybridization is realized with a result of hybridization of reference samples only containing the target sequence to be detected.

16. A computer readable recording medium having recorded thereon a program for performing the step a) of a method according any of claims 1 to 9.

17. A method of manufacturing a microarray for the detection of a target sequence, the method comprising:

preparing a matched probe and a mismatched probe according to the method of any one of claims 1 to 9; and fixing the matched probe and the mismatched probe onto a substrate.


**Patentansprüche**

1. Verfahren zum Herstellen von Sonden zum Detektieren einer Zielsequenz, wobei das Verfahren umfasst:

a. Entwerfen einer gepaarten Sonde und/oder fehlgepaarten Sonde durch:

Auswählen einer Verankerungsstelle in der Zielsequenz, wobei die Verankerungsstelle ein Nukleotid in der Zielsequenz ist, welches sich von dem entsprechenden Nukleotid in einer Nicht-Zielsequenz, die die höchste Sequenzähnlichkeit mit der Zielsequenz hat, unterscheidet;
Auswählen eines ersten Bereichs zum Entwerfen der Sonde, wobei der erste Bereich zum Entwerfen der Sonde ein definierter Bereich in der Zielsequenz ist, der definierte Bereich die Verankerungsstelle enthält, wobei der erste Bereich zum Entwerfen der Sonde so definiert ist, dass alle Sonden entwerfbar sind als an den erste Bereich zum Entwerfen der Sonde bindend und eine definierte Länge, die die Verankerungsstelle enthält, besitzend, wobei der erste Bereich zum Entwerfen der Sonde durch Formel 1 dargestellt wird:

$$<\text{Formel 1}>$$

$$i\text{-}n\text{+}1 \leq \text{erster Bereich zum Entwerfen der Sonde} \leq i\text{+}n\text{-}1$$

wobei i eine Verankerungsstelle ist und n die Länge der Sonde ist;
Auswählen einer Sondenauswahlstelle, wobei die Sondenauswahlstelle ein Nukleotid in der Zielsequenz ist, welche sich von dem entsprechenden Nukleotid in einer der Nicht-Zielsequenzen unterscheidet, wobei die Sondenauswahlstelle in dem ersten Bereich zum Entwerfen der Sonde enthalten ist, wobei die Sondenauswahlstellen in einem zweiten Bereich zum Entwerfen der Sonde enthalten sind, und wobei der zweite Bereich zum Entwerfen der Sonde durch Formel 2 dargestellt wird, wenn die Länge der Sonde eine ungerade Zahl ist, und durch Formel 3 dargestellt wird, wenn die Länge der Sonde eine gerade Zahl ist:

<Formel 2>

i-(n-1)/2 ≤ zweiter Bereich zum Entwerfen der Sonde ≤ i+(n-1)/2

<Formel 3>

i-n/2+1 ≤ zweiter Bereich zum Entwerfen der Sonde ≤ i+n/2-1,

wobei i eine Verankerungsstelle ist und n die Länge der Sonde ist; und

Auswählen einer Sonde umfassend (i) Auswählen einer vollständig gepaarten Sonde, wobei die vollständig gepaarte Sonde die Sondenauswahlstelle in der Mitte davon enthält und die vollständig gepaarte Sonde eine Sequenz besitzt, die vollständige Hybridisierung der vollständig gepaarten Sonde mit der Zielsequenz ermöglicht; und (ii) Auswählen einer absichtlich fehlgepaarten Sonde einschließlich der Sondenauswahlstelle an einer ersten Stelle, einem Nukleotid, das mit dem entsprechenden Nukleotid in der Zielsequenz an einer zweiten Stelle nicht hybridisieren kann, und die absichtlich fehlgepaarte Sonde besitzt eine Sequenz, die mit der Ausnahme der zweiten Stelle, vollständige Hybridisierung der absichtlich fehlgepaarten Sonde mit der Zielsequenz ermöglicht; und

b. Synthetisieren oder Isolieren der gepaarten Sonde und fehlgepaarten Sonde wie gestaltet.

2. Verfahren nach Anspruch 1, wobei, wenn es eine Nicht-Zielsequenz, die die höchste Sequenzähnlichkeit mit der Zielsequenz hat, gibt und wenn es 2 oder mehr mögliche Verankerungsstellen in der Zielsequenz in Bezug auf die genannte eine Nicht-Zielsequenz gibt, die Verankerungsstelle eine der genannten 2 oder mehr möglichen Verankerungsstellen ist.

3. Verfahren nach Anspruch 1, wobei, wenn 2 oder mehr Nicht-Zielsequenzen die gleiche höchste Sequenzähnlichkeit mit der Zielsequenz haben, das Nukleotid in der Zielsequenz, das sich von der höchsten Anzahl der entsprechenden Nukleotide in den 2 oder mehr Nicht-Zielsequenzen mit der gleichen höchsten Ähnlichkeit mit der Zielsequenz unterscheidet, als Verankerungsstelle ausgewählt wird.

4. Verfahren nach Anspruch 1, wobei, wenn 2 oder mehr Nicht-Zielsequenzen die gleiche höchste Sequenzähnlichkeit mit der Zielsequenz haben und wenn 2 oder mehr Nukleotide in der Zielsequenz unterschiedlich sind von der gleichen höchsten Anzahl von entsprechenden Nukleotiden in den 2 oder mehr Nicht-Zielsequenzen, die die gleiche höchste Ähnlichkeit mit der Zielsequenz haben, die Verankerungsstelle in der Zielsequenz eine der genannten 2 oder mehr Nukleotide ist.

5. Verfahren nach einem der Ansprüche 1 bis 4, wobei die Mitte der vollständig gepaarten Sonde bei (n+1)/2 ist, wenn die Länge n der vollständig gepaarten Sonde eine ungerade Zahl ist, und die Mitte der vollständig gepaarten Sonde bei einem von n/2 und n/2+1 ist, wenn die Länge n der vollständig gepaarten Sonde eine gerade Zahl ist.

6. Verfahren nach einem der Ansprüche 1 bis 5, wobei die erste Stelle bei 1/3 der absichtlich fehlgepaarten Sonde ist und die zweite Stelle bei 2/3 der absichtlich fehlgepaarten Sonde ist.

7. Verfahren nach Anspruch 6, wobei 1/3 und 2/3 der absichtlich fehlgepaarten Sonde bei m/3 bzw. 2m/3+1 sind, wenn die Länge m der absichtlich fehlgepaarten Sonde ein Vielfaches von 3 ist, absteigende ganze Zahl von m/3 bzw. (absteigende ganze Zahl von m/3)x$_2$+1, oder (absteigende ganze Zahl von m/3)+1 bzw. (absteigende ganze Zahl von m/3)×2+2, wenn die Länge m der absichtlich fehlgepaarten Sonde ein Vielfaches von 3+1 ist, und (absteigende ganze Zahl von m/3)+1 bzw. (absteigende ganze Zahl von m/3x2+2, wenn die Länge m der absichtlich fehlgepaarten Sonde ein Vielfaches von 3+2 ist.

8. Verfahren nach einem der Ansprüche 1 bis 7, wobei die absichtlich fehlgepaarte Sonde länger als die vollständig gepaarte Sonde ist.

9. Verfahren nach Anspruch 8, wobei die Länge n der vollständig gepaarten Sonde 17 bis 25 Basen beträgt und die Länge m der absichtlich fehlgepaarten Sonde 25 bis 36 Basen beträgt.

10. Verfahren zum Nachweis einer Zielsequenz, wobei das Verfahren umfasst:

Herstellen von Sonden zum Detektieren einer Zielsequenz nach einem der in den Ansprüchen 1 bis 9 aufgeführten Verfahren, wobei der Schritt des Auswählens einer Sonde das Auswählen einer vollständig gepaarten Sonde und Auswählen einer absichtlich fehlgepaarten Sonde umfasst;
Inkontaktbringen einer Reaktionsprobe mit der vollständig gepaarten Sonde und der absichtlich fehlgepaarten Sonde; und
Bestimmen einer Hybridisierungsreaktion zwischen den Sonden und den Zielsequenzen.

11. Verfahren nach Anspruch 10, ferner umfassend das Herstellen eines Mikroarrays nach Auswählen der vollständig gepaarten Sonde und der absichtlich fehlgepaarten Sonde, wobei die vollständig gepaarte Sonde und die absichtlich fehlgepaarte Sonde auf einem Substrat fixiert sind.

12. Verfahren nach Anspruch 10 oder 11, weiter umfassend einen Waschschritt nach dem Inkontaktbringen der Reaktionsprobe mit den Sonden, wobei die Reaktionsprobe im wesentlichen von den Sonden entfernt wird, um eine unspezifische Reaktion zu vermeiden.

13. Verfahren nach einem der Ansprüche 10 bis 12, wobei das Bestimmen der Hybridisierungsreaktion durch Detektieren der Fluoreszenzintensität und Bestimmen, dass die Hybridisierung realisiert ist, wenn die detektierte Intensität über einem vorgegebenen Wert ist, durchgeführt wird.

14. Verfahren nach einem der Ansprüche 10 bis 13, ferner umfassend das Bestimmen der Anwesenheit der Zielsequenz nach der Bestimmung der Hybridisierungsreaktion, wobei die Anwesenheit der Zielsequenz unter Verwenden des Ergebnisses, das zeigt, ob eine Hybridisierung oder keine Hybridisierung realisiert ist, bestimmt wird.

15. Verfahren nach Anspruch 14, wobei das Bestimmen der Anwesenheit der Ziel-Sequenz durch Vergleichen des Ergebnisses, das zeigt, ob eine Hybridisierung oder keine Hybridisierung realisiert ist, mit einem Ergebnis der Hybridisierung von Referenzproben, die nur die zu detektierende Zielsequenz enthalten, durchgeführt wird.

16. Computerlesbares Aufzeichnungsmedium mit einem darauf aufgezeichneten Programm zum Ausführen des Schritts a) eines Verfahrens nach einem der Ansprüche 1 bis 9.

17. Verfahren zur Herstellung eines Mikroarrays zum Nachweis einer Zielsequenz, wobei das Verfahren umfasst:

Herstellen einer gepaarten Sonde und einer fehlgepaarten Sonde nach dem Verfahren nach einem der Ansprüche 1 bis 9; und
Befestigen der gepaarten Sonde und der fehlgepaarten Sonde auf einem Substrat.

**Revendications**

1. Une méthode de préparation de sonde pour détecter une séquence cible comprenant:

a. la conception d'une sonde « matched » et/ou d'une sonde « mismatched » par :

la sélection d'un emplacement d'ancrage dans la séquence cible, dans laquelle l'emplacement d'ancrage est un nucléotide dans la séquence cible qui est différent du nucléotide correspondant dans une séquence non cible ayant la similarité de séquence la plus grande avec la séquence cible ;
la sélection d'une première région de conception d'une sonde, dans laquelle la première région de conception d'une sonde est une région définie dans la séquence cible, où la région définie inclut un emplacement d'ancrage, dans laquelle la première région de conception d'une sonde est définie telle que toutes les sondes faites pour se lier à la première région de conception d'une sonde et ayant une longueur définie inclut un emplacement d'ancrage, dans laquelle la première région de conception d'une sonde est représentée par la Formule 1 :

< Formule 1>

i-n+1 ≤ première région de conception d'une sonde ≤ i+n-1

dans laquelle, i représente un emplacement d'ancrage et n représente la longueur de la sonde ;
la sélection d'un emplacement de choix d'une sonde, dans laquelle l'emplacement de choix d'une sonde est un nucléotide dans la séquence cible qui est différent du nucléotide correspondant dans une quelconque des séquences non cibles, dans laquelle l'emplacement de choix d'une sonde est inclus dans la première région de conception d'une sonde, dans laquelle les emplacements de choix d'une sonde sont inclus dans une deuxième région de conception d'une sonde, et dans laquelle la deuxième région de conception d'une sonde est représentée par la Formule 2 quand la longueur de la sonde est un nombre impair, et est représentée par la Formule 3 quand la longueur de la sonde est un nombre pair :

< Formule 2>

i-(n-1)/2 ≤ deuxième région de conception d'une sonde ≤ i+(n-1)/2

< Formule 3>

i-n/2+1 ≤ deuxième région de conception d'une sonde ≤ i+n/2-1,

dans laquelle, i représente un emplacement d'ancrage et n représente la longueur de la sonde ; et
la sélection d'une sonde comprenant (i) la sélection d'une sonde « matched » complète, dans laquelle la sonde « matched » complète inclut l'emplacement de choix d'une sonde au centre de celle-ci et où la sonde « matched » complète a une séquence permettant l'hybridation complète de la sonde complètement adaptée avec la séquence cible; et (ii) la sélection de la sonde « mismatched » correspondante incluant l'emplacement de choix d'une sonde à un premier emplacement, un nucléotide qui ne peut s'hybrider avec le nucléotide correspondant dans la séquence cible à un deuxième emplacement, et où la sonde « mismatched » correspondante a une séquence permettant, sauf pour le deuxième emplacement, de terminer l'hybridation de la sonde « mismatched » avec la séquence cible ; et

b. la synthèse ou l'isolation de la sonde "matched" et de la sonde "mismatched" telles que fabriquées.

2. La méthode selon la revendication 1, dans laquelle, si il y a une séquence non cible ayant la même similarité la plus élevée avec la séquence cible et si il y a 2 emplacements d'ancrage possibles ou plus dans la séquence cible par rapport à ladite une séquence non cible, l'emplacement d'ancrage est l'un quelconque desdits deux ou plusieurs emplacements possibles d'ancrage.

3. La méthode selon la revendication 1, dans laquelle, si 2 séquences non cibles ou plus ont la même similarité la plus élevée avec la séquence cible, le nucléotide dans la séquence cible, qui est différent du nombre le plus élevé de nucléotides correspondants dans les 2 séquences non cibles ou plus ayant la même similarité la plus élevée avec la séquence cible, est sélectionné en tant qu'emplacement d'ancrage.

4. La méthode selon la revendication 1, dans laquelle, si 2 séquences non cibles ou plus ont la même similarité la plus élevée avec la séquence cible et si 2 ou plusieurs nucléotides dans la séquence cible sont différents du même nombre le plus élevé de nucléotides correspondants dans 2 séquences non cibles ou plus ayant la même similarité la plus élevée avec la séquence cible, l'emplacement d'ancrage dans la séquence cible est un quelconque desdits 2 nucléotides ou plus.

5. La méthode selon une quelconque des revendications 1 à 4, dans laquelle le centre de la sonde "matched" complète est de (n+1)/2 th quand la longueur n de la sonde "matched" complète est un nombre impair, et le centre de la sonde "matched" complète est d'une quelconque de n/2 th et n/2+1 th quand la longueur n de la sonde "matched" complète est un nombre pair.

6. La méthode selon une quelconque des revendications 1 à 5, dans laquelle le premier emplacement est au 1/3 de la sonde "mismatched" correspondante, et le deuxième emplacement est au 2/3 de la sonde "mismatched" correspondante.

7. La méthode selon la revendication 6, dans laquelle les 1/3 et 2/3 de la sonde "mismatched" correspondante sont de m/3 th et 2m/3+1 th, respectivement, quand la longueur m de la sonde "mismatched" correspondante est un multiple de 3, entier décroissant de m/3 et (entier décroissant de m/3)×2+1 th, respectivement, ou (entier décroissant de m/3)+1 st et (entier décroissant de m/3)×2+2 th, respectivement, quand la longueur m de la sonde "mismatched" correspondante est un multiple de 3+1, et (entier décroissant de m/3)+1 st et (entier décroissant de m/3×2+2 nd, respectivement, quand la longueur m de la sonde "mismatched" correspondante est un multiple de 3+2.

8. La méthode selon une quelconque des revendications 1 à 7, dans laquelle la sonde "mismatched" correspondante est plus longue que la sonde "matched" complète.

9. La méthode selon la revendication 8, dans laquelle la longueur n de la sonde "matched" complète est de 17 à 25 bases et la longueur m de la sonde "mismatched" correspondante est 25 à 36 bases.

10. Une méthode de détection de séquence cible, la méthode comprenant:

la préparation des sondes pour la détection d'une séquence cible selon une quelconque des méthodes telles que dans les revendications 1 à 9, dans laquelle l'étape de sélection d'une sonde comprend la sélection d'une sonde « matched » complète et la sélection d'une sonde « mismatched » correspondante ;
la mise en contact d'un échantillon de réaction avec la sonde « matched » complete et la sonde « mismatched » corrrespondante; et
la détermination d'une réaction d'hybridation entre les sondes et les séquences cibles.

11. La méthode selon la revendication 10, comprend en outré la fabrication d'une puce à ADN après la sélection d'une sonde "matched" complète et la sonde "mismatched" correspondante, dans laquelle la sonde "matched" complète et la sonde "mismatched" correspondante sont fixées sur un substrat.

12. La méthode selon la revendication 10 ou 11, comprenant en outre une étape de lavage après avoir mis en contact l'échantillon de réaction avec les sondes, dans laquelle l'échantillon de réaction est sensiblement retiré de la sonde pour éliminer une réaction non spécifique.

13. La méthode selon une quelconque des revendications 10 à 12, dans laquelle la reaction d'hybridation est exécutée en détectant l'intensité de fluorescence et en déterminant si l'hybridation est réalisée quand l'intensité détectée est supérieure à une valeur prédéterminée.

14. La méthode selon une quelconque des revendications 10 à 13, comprend en outré la détermination de la séquence cible après la détermination de la réaction d'hybridation, dans laquelle la présence de la séquence cible est détermine en utilisant les résultats indiquant si oui ou non l'hybridation est réalisée.

15. La méthode selon la revendication 14, dans laquelle la détermination de la présence d'une séquence cible est faite en comparant le résultat indiquant si oui ou non l'hybridation est réalisé à la suite de l'hybridation des échantillons de référence contenant uniquement la séquence cible à détecter.

16. Un support d'enregistrement lisible par ordinateur sur lequel est enregistré un programme pour exécuter l'étape a) d'une méthode selon une quelconque des revendications 1 à 9.

17. Procédé de fabrication d'une puce à ADN pour la détection d'une séquence cible, le procédé comprenant:

la préparation d'une sonde "matched" et d'une sonde "mismatched" d'après la méthode selon une quelconque des revendications 1 à 9; et
la fixation de la sonde "matched" et de la sonde "mismatched" sur un substrat.

# FIG. 1

EP 1 889 924 B1

```
                                          tttcgaaggagacgttgttg — PROBE (SEQUENCE NO. 17)
SEQUENCE NO.1: ctahgagatcgggacgccagtttcgaaggagacgttgttgggatactacccttgtgttat — TARGET
                                                                            SEQUENCE
SEQUENCE NO.2: ctaagagatcgggacgccagtttcgaaggagacgCtgttgggatactacccttgtgttat
SEQUENCE NO.3: cattgaagtcgggacgccagtttcgaCggagGcgttgttgggatactacccttgtgttat
SEQUENCE NO.4: cattgacttcgggacgccagtttcgaaTgagGcgttgttgggatactacccttgtgttat
SEQUENCE NO.5: ctawgaratcgggacgccagtttcgaTggagGcgttgttgggatactacccttgtgttat    NON-TARGET
SEQUENCE NO.6: ctatgaaatcgggacgctagtttcgGTggagGcgttgttgggatactacccttgtgttat    SEQUENCES
SEQUENCE NO.7: ctacgagatcgggacgccagtttcgaTggagGcgCtgttgggatactacccttgtgttat
SEQUENCE NO.8: ctaggaatcatgcacgccagtGtATGaggagGcgCtgttgggatactacccttgctgtat
```

# FIG. 2

```
                                    tcgggacgccagtttcgaag —— PROBE 1      ⎫
                                     cgggacgccagtttcgaagg —— PROBE 2      ⎪
                                            .  .  .                       ⎪
                                                              .           ⎬ PROBES
                                  gagacgttgttgggatacta —— PROBE 21        ⎪
                                   agacgttgttgggatactac —— PROBE 22       ⎭
                                    gacgttgttgggatactacc —— PROBE 23
```

| | |
|---|---|
| SEQUENCE NO.1: ctahgagatcgggacgccagtttcgaaggagacgttgttgggatactaccttgtgttat | —— TARGET SEQUENCE |
| SEQUENCE NO.2: ctaagagatcgggacgccagtttcgaaggagacgCtgttgggatactaccttgtgttat | ⎫ |
| SEQUENCE NO.3: cattgaagtcgggacgccagtttcgaCggagGcgttgttgggatactaccttgtgttat | ⎪ |
| SEQUENCE NO.4: cattgacttcgggacgccagtttcgaaTgagGcgttgttgggatactaccttgtgttat | ⎬ NON-TARGET |
| SEQUENCE NO.5: ctawgaratcgggacgccagtttcgaTggagGcgttgttgggatactaccttgtgttat | SEQUENCES |
| SEQUENCE NO.6: ctatgaaatcgggacgctagtttcgGTggagGcgttgttgggatactaccttgtgttat | ⎪ |
| SEQUENCE NO.7: ctacgagatcgggacgccagtttcgaTggagGcgCtgttgggatactaccttgtgttat | ⎪ |
| SEQUENCE NO.8: ctaggaatcatgcacgccagtGtATGaggagGcgCtgttgggatactaccttgctgtat | ⎭ |

# FIG. 3

```
                    ( START )
                        │
                        ▼
    ┌───────────────────────────────────────┐
    │  SELECTING  AN ANCHORING LOCATION      │── 31
    └───────────────────────────────────────┘
                        │
                        ▼
    ┌───────────────────────────────────────┐
    │       SELECTING A FIRST PROBE          │── 32
    │            DESIGNING REGION            │
    └───────────────────────────────────────┘
                        │
                        ▼
    ┌───────────────────────────────────────┐
    │  SELECTING PROBE SELECTION LOCATIONS   │── 33
    └───────────────────────────────────────┘
                        │
                        ▼
    ┌───────────────────────────────────────┐
    │  SELECTING A COMPLETE MATCHED PROBE    │── 34
    └───────────────────────────────────────┘
                        │
                        ▼
    ┌───────────────────────────────────────┐
    │        SELECTING A INTENDED            │── 35
    │          MISMATCHED PROBE              │
    └───────────────────────────────────────┘
                        │
                        ▼
                    (  END  )
```

# FIG. 4A

ANCHORING LOCATION

SEQUENCE NO.1: ctahgagatcgggacgccagtttcgaaggagacgttgttgggatactacccttgtgttat — TARGET SEQUENCE

SEQUENCE NO.2: ctaagagatcgggacgccagtttcgaaggagacgCtgttgggatactacccttgtgttat

SEQUENCE NO.3: cattgaagtcgggacgccagtttcgaCggagGcgttgttgggatactacccttgtgttat

SEQUENCE NO.4: cattgacttcgcgacgccagtttcgaaTgagGcgttgttgcggatactacccttgtgttat

SEQUENCE NO.5: ctawgaratcgggacgccagtttcgaTggagGcgttgttgcggatactacccttgtgttat

SEQUENCE NO.6: ctatgaaatcgggacgctagtLtcgGTggagGcgttgttgggatactacccttgtgttat

SEQUENCE NO.7: ctacgagatcgggacgccagtttcgaTggagGcgCtgttgggatactaccctcgtgttat

SEQUENCE NO.8: ctaggaatcatgcacgccagtGtATGaggagGcgCtgttgggatactacccttgctgtat

NON-TARGET SEQUENCES

## FIG. 4B

ANCHORING LOCATION 1    ANCHORING LOCATION 2

TARGET SEQUENCE

NON-TARGET SEQUENCE 1
NON-TARGET SEQUENCE 2
NON-TARGET SEQUENCE 3
NON-TARGET SEQUENCE 4
NON-TARGET SEQUENCE 5
NON-TARGET SEQUENCE 6
NON-TARGET SEQUENCE 7
NON-TARGET SEQUENCE 8

## FIG. 4C

ANCHORING LOCATION

TARGET SEQUENCE

NON-TARGET SEQUENCE 1
NON-TARGET SEQUENCE 2
NON-TARGET SEQUENCE 3
NON-TARGET SEQUENCE 4
NON-TARGET SEQUENCE 5
NON-TARGET SEQUENCE 6
NON-TARGET SEQUENCE 7
NON-TARGET SEQUENCE 8

# FIG. 5

```
                                    ttgttgggatactaccc — PROBE 1
                                  gttgttgggatactacc — PROBE 2
                                        :
                            tttcgaaggagacgttg —PROBE 15
                           gtttcgaaggagacgtt —PROBE 16
                          agtttcgaaggagacgt —PROBE 17
```

FIRST PROBE DESIGNING REGION (WHEN n=17)

i-n+1 | ANCHORING LOCATION | i+n-1

SEQUENCE NO.1: ctahgagatcgggacgccagtttcgaaggagacgttgttgggatactacccttgtgttat — TARGET SEQUENCE

SEQUENCE NO.2: ctaagagatcgggacgccagtttcgaaggagacgCtgttgggatactacccttgtgttat

SEQUENCE NO.3: cattcaagtcgggacgccagtttcgaCggagGcgttgttgggatactacccttgtgttat

SEQUENCE NO.4: cattgacttcgggacgccagtttcgaaTgagGcgttgttgggatactacccttgtgttat

SEQUENCE NO.5: ctawgaratcgggacgccagtttcgaTggagGcgttgttgggatactacccttgtgttat  NON-TARGET SEQUENCES

SEQUENCE NO.6: ctatgaaatcgggacgctagtttcgGTggagGcgttgttgggatactacccttgtgttat

SEQUENCE NO.7: ctacgagatcgggacgccagtttcgaTggagGcgCtgttgggatactacccttgtgttat

SEQUENCE NO.8: ctaggaatcatgcacgccagtGtATGaggagGcgCtgttgggatactacccttgctgtat

EP 1 889 924 B1

# FIG. 6

ANCHORING LOCATION

|1 2  3 4▼  PROBE SELECTION
▼▼  ▼ ▼     LOCATION

SEQUENCE NO.1: ctahgagatcgggacgccagtttcgaaggagacgttgttggga actaccc tgtgttat — TARGET SEQUENCE

SEQUENCE NO.2: ctaagagatcgggacgccagtttcgaaggagacgCtgttggga actaccc tgtgttat ⎫
SEQUENCE NO.3: cattgaactcgggacgccagtttcgaCggagGcgttgttggga actaccc tgtgttat │
SEQUENCE NO.4: cattgacttcgggacgccagtttcgaaTgagGcgttgttggga actaccc tgtgttat │
SEQUENCE NO.5: ctawgaratcgggacgccactttcgaTggagGcgttgttggga actaccc tgtgttat ⎬ NON-TARGET SEQUENCES
SEQUENCE NO.6: ctatgaaatcgggacgctactttcgGTggagGcgttgttcgga actaccc tgtgttat │
SEQUENCE NO.7: ctacgagatcgggacgccagtttcgaTggagGcgCtgttggga actaccc tgtgttat │
SEQUENCE NO.8: ctaggaatcatgcacgccagtGtATGaggagGcgCtgttggga actaccc tgctgtat ⎭

SECOND PROBE DESIGNING REGION

FIRST PROBE DESIGNING REGION

SEQUENCE NO.9: agtttcgaAggagacgt            pm#1 (n=17)
SEQUENCE NO.10: agtttcgaAggagacgtAgttggga    mm#1 (m=25)

SEQUENCE NO.11: gtttcgaaGgagacgtt            pm#2
SEQUENCE NO.12: gtttcgaaGgagacgttCttgggat    mm#2

SEQUENCE NO.13: cgaaggagAcgttgttg            pm#3
SEQUENCE NO.14: cgaaggagAcgttgttgCgatacta    mm#3

┌─ CENTER
SEQUENCE NO.15: aggagacgTtgttggga            pm#4
SEQUENCE NO.16: aggagacgTtgttgggaAactaccc    mm#4

1/3 LOCATION      2/3 LOCATION

# FIG. 7

START

SELECTING AN ANCHORING LOCATION — 70

SELECTING A FIRST PROBE
DESIGNING REGION — 71

SELECTING PROBE SELECTION LOCATIONS — 72

SELECTING A COMPLETE MATCHED PROBE — 73

SELECTING A INTENDED
MISMATCHED PROBE — 74

MANUFACTURING A MICROARRAY — 75

PROVIDING A REACTION SAMPLE — 76

WASHING — 77

CONFIRMING HYBRIDIZATION REACTION — 78

DETERMINING THE PRESENCE
OF THE TARGET SEQUENCE — 79

END

# FIG. 8

[REFERENCE DATA]

SAMPLE ONLY
INCLUDING ⟶
TARGET SEQUENCE

[EXPERIMENTAL DATA]

SAMPLE 1 ⟶     : TARGET SEQUENCE

SAMPLE 2 ⟶     : TARGET SEQUENCE + α

SAMPLE 3 ⟶     : TARGET SEQUENCE X

30

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0111082 A

- KR 1020060077826

**Non-patent literature cited in the description**

- **BODLEY TROUP ; MARTIN ; MCMILLAN ; HORTON.** *Proceedings of the 2005 IEEE Computational Systems Bioinformatics Conferences Workshops,* 2005
- **GUO, Z. et al.** Enhanced Discrimination of Single Nucleotide Polymorphisms by Artificial Mismatch Hybridization. *Nature Biotechnology,* April 1997, vol. 15, 331-335

- High-density microarray of small-subunit ribosomal DNA probes. **WILSON, KENNETH et al.** Applied and Environmental Microbiology. American Society for Microbiology, 01 May 2002, vol. 68, 2535-2541